# EUROPEAN PATENT APPLICATION

(11) **EP 1 593 374 A1**
(43) Date of publication of application: **09.11.2005**
(21) Application number: 04010920.9
(22) Date of filing: 07.05.2004
(51) Int. Cl.: A61K 9/00

(54) **Remote control release of encapsulated material**

(71) Applicant: Max-Planck-Gesellschaft zur Förderung der Wissenschaften e.V., 80539 München (DE)
(72) Inventor: Antipov, Alexei., 12450 Berlin (DE); Mamedov, Arif., Stillwater, OK 74075 (US); Sukhorukov, Gleb., 14471 Potsdam (DE); Möhwald, Helmuth., 55411 Bingen (DE); Kotov, Nicholas., Ypsilanti, Michigan 48198 (US); Skirtach, Andre., 14532 Berlin-Kleinmachnow (DE)
(74) Representative: Dey, Michael

(57) **Abstract**

The present invention relates to a method for remote control release of encapsulated substances by external forces such as ultrasound or light.

## Description

The present invention relates to a method for remote control release of encapsulated substances by external forces such as ultrasound or light.

Hollow polymer capsules prepared utilizing layer-by-layer assembly technique become an object of interest in different research areas such as catalysis, biotechnology, biosensing etc. The possibility to load various materials into the empty capsules provides an opportunity to employ them in medical diagnostic and treatment purposes. However, to achieve the maximum success in such a task, it is important that capsules can release enclosed substances, e.g. a drug, as close as possible to the tested or treated area. One of the possible solutions is to functionalize the capsule surface with a "trigger" which will activate drug release once the capsule is in vicinity of a tested or treated area. This "trigger" can be activated from inside of the body. The inside activation can be started e.g. by specific molecules, compounds or environmental conditions present only in the tested or treated area.

However, such release cannot be controlled from outside the body and, thus, release of the active substance at a desired time or a desired site is possible only under certain circumstances.

Therefore, it was an object of the invention to provide an improved method for release and, in particular, for sustained release of encapsulated compounds.
According to the invention this object is achieved by a method for remote control release of a substance from a capsule, wherein (i) a capsule containing a substance to be released is provided, (ii) the capsule shell is rendered permeable to the substance by forces exerted by a remote external source such that (iii) the substance is released from the capsule.

According to the invention release of substances enclosed in capsules can be effected at any time and any site by remote control release, irrespective of the environment. This makes it possible, for example, to release active substances inserted into a body in encapsulated form by external activation. According to the invention it is not necessary to specifically functionalize the capsule wall, e.g. in order to target the capsules into a specific tissue or specific organs, or to render the capsules specific for particular diseases, e.g. different types of cancer. Rather can a single capsule type be used, whereby only the forces required for release, which originate from a remote external source, are radiated onto the desired site of action at the desired point in time.

The remote control release or activation of encapsulated substances according to the invention is effected by irradiation with forces exerted by a remote external source. Thus, the activation source is remote from the capsules, upon which the exerted forces act, in particular, at least 1 mm, more preferably, at least 1 cm, even more preferably, at least 5 cm, and most preferably, at least 10 cm. However, it is also possible to effect activation at great distances, e.g. at least 1 m. No compounds or molecules are required to exert the forces, so the remote external source is not in direct contact with the capsules. Said forces, for example, can be ultrasound or/and light.

According to the invention, a capsule containing a substance to be released is provided in a first step (i). The substance thereby is preferably positioned in the interior of the capsule, however, it can also be embedded in the shell of the capsule or/and attached to the shell of the capsule.

Capsules obtainable by layer-by-layer assembly of alternately charged polyelectrolytes and/or nanoparticles are especially preferred. Such capsules obtained by layer-by-layer (LbL) technology can be easily produced in defined size, with defined shell thickness and defined functionality of the shells. For providing a capsule containing a substance to be released the substance can be provided optionally together with a matrix and then encapsulated by alternately applying polyelectrolyte and/or nanoparticle layers. Thereby, positively and negatively charged layers are applied alternately However, it is also possible to first produce a hollow capsule by the layer-by-layer technology by applying the layers onto a core template and then the dissolving the core template and removing same from the capsule. Such hollow capsules then can be subsquently charged with substances. For charging, permeability of the capsule shell can be varied, e.g. by modifying the pH or the salt content of the surrounding solution. However, it is also possible to insert the substance to be released in the shell, e.g. embed it between two layers of the shell or provide it as a component of one of the layers. In another embodiment the substance to be released is attached to the shell of the capsule, e.g. to the outer side or inner side of the capsule shell.

According to the invention the substance to be released can be chosen arbitrarily, whereby, in particular, sensitive substances can be used as well. Especially preferably, substances selected from pharmaceutical agents, biomolecules, catalytically active molecules such as enzymes, biosensing molecules or other bioactive compounds are used.

Particularly preferably active substances are used which are medical diagnostic or therapeutic agents.

In a next step (ii) according to the invention, the capsule shell is rendered permeable to the substance by forces exerted by remote external source. Thereby it is sufficient according to the invention that the substance can emerge through the capsule wall. However, it is also possible to open the capsule shell or completely destroy the capsules to release the encapsulated substance.

In an especially preferred embodiment the remote release is caused by ultrasound activation, whereby continuous sonication or/and pulse sonication can be applied. In the case of continuous sonication the impact of the external force, for example, can be from 1 min to 10 h, more preferably, 5 min to 1 h. Typical power is in the range of from 100-2000 W, more preferably 500-1000 W. Preferably, pulse sonication is carried out, e.g. with pulses having a length of from 0.1 to 10 sec, in particular, from 0.5 to 2 sec, and corresponding pauses of from 0.1 to 10 sec, preferably from 0.5 to 2 sec.

In another preferred embodiment, the remote release is caused by light irradiation, especially by light irradiation selected from continuous wave irradiation or/and pulsed irradiation. Preferably light having near-ultraviolet wavelength, e.g. from 100-400 nm, in particular, 200-400 nm, visible wavelength, e.g. from 400-750 nm and/or infrared wavelength, e.g. from 750-1500 nm, in particular from 750-1000 nm, is used. Light irradiation is preferably effected by a laser, e.g. a short-pulsed laser at nanosecond or femtosecond range or a CW laser. The light can be focussed on the capsules by an optical system, e.g. a microscope objective or a lens. Preferably, light having a wavelength of from 700 to 1000 nm is irradiated, i.e. light in the near-infrared range. In said range absorption of biological compounds is minimal, so irradiation and selective activation of the capsules, e.g. in a body, is possible without body tissue taking up light and possibly being damaged.

Light irradation to specific sites can also be effected via fiber optics.

Another essential advantage of the invention is that the enclosed substances and the exerted forces can be chosen so that the capsule wall is made permeable, however, the exerted forces themselves have no impact on the substance to be released, in particular, that they do not change or destroy said substances. To this end, the capsule shells preferably are designed in such a way that the capsule wall is capable of absorbing the exerted forces, with the enclosed substances not being impaired. This can be achieved by the election of the remote energy source as well as by the election of suitable intensities of the acting forces. For example, it is preferred to produce capsules, the capsule wall components of which have an absorption spectrum which absorbs the irradiated light, and at the same time the laser light intensities are elected so as to avoid any negative impact on the enclosed substances. Especially preferably, the capsule wall is doped with material sensitive and/or susceptible to the force exerted by the external source. For example, by introducing dye molecules or metal particles, e.g. silver metal particles, capsule shells can be produced which can be opened selectively by external excitation with light. The dyes and the light can be coordinated thereby. In the case of ultrasound excitation, the capsules are preferably doped with magnetite nanoparticles, nanodiamonds, silica nanoparticles, ceramic nanoparticles or similar particles which absorb the ultrasound energy.

The capsules also can be doped with polymers susceptible to the exerted force.

According to the invention it is also possible to successfully monitor remote control release, e.g. using labels, in particular, fluorescence labels. For example, the distribution of the encapsulated substance in the capsules and release thereof, respectively, can be monitored by means of fluorescence microscopy using fluorescing labels.

The invention further relates to a capsule for remote control release comprising a capsule shell obtainable by layer-by-layer assembly of alternately charged polyelectrolytes and/or nanoparticles, wherein the shell is doped with material sensitive and/or susceptible to a force exerted by an remote external source, which force renders the capsule shell wall permeable to a substance to be released.

The capsules of the invention which are obtainable by the layer-by-layer technology have a shell doped with material which enables opening of the shell by exerting forces caused by remote control. The shell thereby preferably has silver nanoparticles, dyes, magnetite nanoparticles, nanodiamonds or silica nanoparticles, which are activated by light irradiation and ultrsound, respectively, thereby enabling opening of the capsules or destruction thereof, while the substance to be released remains intact.

The remote activation of encapsulated substance release according to the invention allows to transport encapsulated substances and release thereof at various target sites, so the method can be used for the most various applications. Especially preferably, the method is applied in diagnosis or therapy, whereby the encapsulated substances are incorporated into the body and then released at the desired site, e.g. in a desired organ or near a diagnosed focus of disease, especially a tumor. In this particular embodiment concerning diagnostic or therapeutic use, the method of the invention is also suitable for in vitro application.

The method of the invention further can be used for catalysis, whereby the encapsulated substance is a catalyst which in that case is transported to the desired site of action and can be released at a desired time. Further possible applications are in biotechnology, in biosensing and/or in medicine.

The invention is further illustrated by the attached Figures and the Examples.
- Fig. 1: shows the destruction of capsules doped with Ag nanoparticles by light irradiation with a CW laser source in the form of a LD having an intensity of up to 75 mw and a wavelength of 750 nm. In Fig. 1(a) the capsule under error is intact, while in Fig. 1(d) the same capsule is shown destroyed.
- Fig. 2: shows capsules having the composition (PSS/PAH)₈, loaded with fluorescence-labeled PAH, modified with magnetite (A) or silver (B) nanoparticles after 1 h of continuous sonication.
- Fig. 3: shows confocal micrographs of non-modified (A) or magnetitemodified (B) capsules having the shell composition (PSS/PAH)₈, loaded with fluorescein-labeled PAH after 6 min of pulse sonication.
- Fig. 4: shows optical images of non-modified (A) or magnetite-modified (B) (PSS/PAH)₈ capsules, loaded with precipitated fluorescein after 6 min of pulse sonication. The solution was excited with a UV lamp emitting light with 366 nm wavelength.
- Fig. 5(A): shows the kinetics of pulse sonication of (PSS/PAH)₂ Ag (PSS/PAH) capsules. Confocal micrographs are taken in transmission mode.
- Fig. 5(B): shows fluoro-spectra of 15 minutes sonication for non-modified (A) and magnetite modified (B) capsules loaded with precipitated fluorescein.
- Fig. 6: shows the kinetics of sonication for non-modified and magnetitemodified capsules loaded with precipitated fluorescein.
- Fig. 7(A): shows typical UV-vis spectra of conversion of 4-NP into 4-AP in the presence of a silver catalyst.
- Fig. 7(B): shows the decrease of Abs₄₀₀ for 4-NP in (1) the absence or presence of (2) non-sonicated or (3) sonicated (PSS/PAH)₂Ag (PSS/PAH)₂ capsules.
- Fig. 8: shows the decrease of Abs₄₀₀ for 4-NP in (1) the absence or presence of (2) non-sonicated and (3) sonicated (PSS/PAH)₈Ag-loaded (PSS/PAH)₂capsules.
- Fig. 9: shows the decrease of Abs₄₀₀ for 4-NP in the presence of (1) non-sonicated and (2) sonicated (PSS/PAH)₈Ag-loaded (PSS/PAH)₂capsules.

### Examples

### Example 1

### Activation of controlled release by light illumination

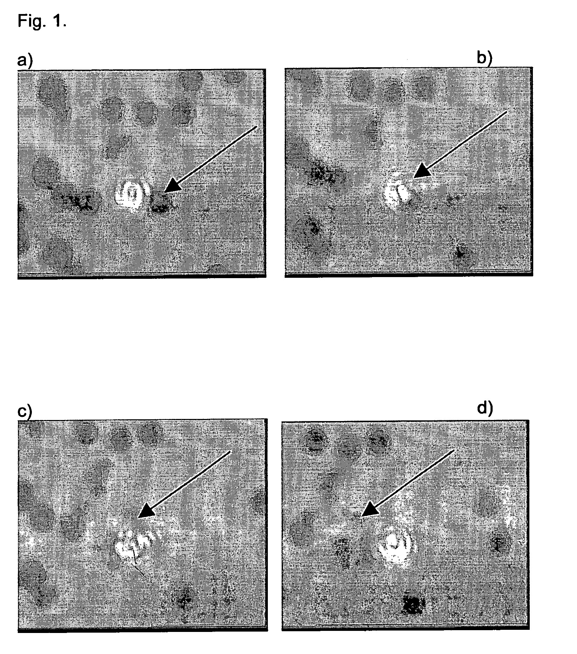

Capsules doped with Ag nanoparticles or doped with Ir806 dye were irradiated with an LD (CW laser source) with an intensity of up to 75 mW and a wavelength of 750 nm. The capsules were destroyed thereby and the content of the capsules was released. Fig. 1 shows the disintegration of a capsule doped with Ag nanoparticles.

### Example 2

### Remote activation of encapsulated substance release by ultrasound

### 2.1 Materials.

### Chemicals:

Sodium poly(styrenesulfonate) (PSS, MW ~ 70,000), poly(allylamine hydrochloride) (PAH, MW ~ 50,000) were obtained from Aldrich. Salts and buffer components were purchased from Roth (Germany). Melamineformaldehyde (MF) slightly crosslinked latexes of 5 µm diameter were purchased from Microparticles GmbH (Germany). All chemicals were used without further purification. Ultra-pure deionized milli-Q water prepared in three-stage purification Milli-Pore system was used in all experiments. Magnetic particles was a gift sample from Humboldt University, nanodiamonds was a gift sample from PlasmaChem GmbH (Germany). Si02 particles of 100-200 nm in diameter were purchased from Sigma-Aldrich Co (Germany).

PAH was labeled with fluorescein marker following the standard procedure. PAH (0.5 g) and FITC (3 mg) were dissolved in borate buffer, pH 9 and mixed together. Two hours of incubation was followed by dialysis against water in the dialysis bags with 25kDa MWCO (Spectrapor, Germany).

### Capsules:

*Polyelectrolyte capsules* were fabricated as follows: Colloidal particles (MF latexes or silvered particles) were incubated with each polyelectrolyte of 5 mM monomer unit concentration in 0.5 M NaCl solution for 15 minutes. Triple washing with water and centrifugation was finishing each adsorption circle.

PAH-loaded capsules were fabricated as described in [1]. FITC-PAH of 2 mg/ml was controlled precipitated with sodium citrate and covered with 8 PSS/PAH layers. After the core dissolution, citric ions were removed from the capsules by dialysis against water for 24 hours. The formed capsules were containing 10 mg/ml FITC-PAH as determined by fluorescence spectroscopy.

*Silver containing capsules* were prepared according to [2]. The capsules were loaded with silver particles by adding [Ag(NH₃)₂]NO₃ to dispersion of nanocapsules and stirring (shaking) for 2 hours. The concentration of silver nitrate in final solution was 0.1 M. Upon completion of silver particles formation two additional bilayers of PSS/PAH have been assembled to reduce chance of contact of silver particles with a surrounded media. The final structure of capsule can be expressed as (PSS/PAH)₈ Ag_{loaded} (PSS/PAH)₂.

Capsules with silver-containing walls were fabricated by means of electroless plating of silver onto the MF particles modified with (PSS/PAH)₂ layers followed by adsorption of two additional PSS/PAH bilayers. The final silver concentration was *25 pg* of silver per capsule.

Fluorescein loaded capsules were fabricated as follows: (PSS/PAH)₄ capsules loaded with 0.1 M (monomer unit concentration) PSS were incubated with 10 mg/ml solution of fluorescein for 5 hours. Capsules have been washed with acetic buffer for 10 minutes and centrifuged out on a microcentrifure at 300 g for 7 minutes. Further experiments with fluorescein-containing capsules were conducted in pH=5.24 acetic buffer prepared from 0.01 M acetic acid and 0.01 M sodium acetate mixed in a ratio 3:7.

### 2.2 Methods.

### Surface modification of capsules.

1) *Magnetite or silica containing capsules* were prepared similarly to the polyelectrolyte ones, with the only difference that instead of the second PSS layer, magnetic or SiO₂ particles were adsorbed.
2) Saturated solution of nanodiamonds has been diluted 100 times with water. SiO₂ nanoparticles have been assembled in presence of 0.1 M NaCl. Stock solution of magnetite nanoparticles has been diluted 40 times. Obtained solution has been diluted in a ratio 1:1 with (a) water for PAH loaded capsules and (b) acetate buffer for capsules, loaded with precipitated fluorescein. Capsules have been washed for 30 minutes (nanodimonds and latex particles) and 10 minutes (magnetite particles) modified to remove unattached nanomaterial after adsorption. For capsules with precipitated fluorescein, the washing step was carried out with acetic buffer.

### 2.3 Destruction of capsules.

*Continuous sonication*. Capsules have been continuously sonicated for 1 hour in a bath sonicator with 750 W power (Bandelin electronics, Germany).

*Pulse sonication*. Capsules have been sonicated in pulse mode with the Bandelin sonoplus HD 200 (Bandelin electronic, Berlin, Germany) in 1/1 cycle (1 sec. pulse/ 1 second pause) using 3mm diameter probe operating at 20 kHz. The total power of the ultrasound was equal to 120 W.

*Confocal micrographs* were taken with Leica TCS SP, equipped with 100x oil immersion objective with numerical aperture of 1.4.
*Optical photographs* were taken with Sony 85MD digital camera. Samples were excited with 366 nm UV lamp.

*Fluorescent spectra* were taken with FluoroMax fluorimenter on the facilities of University of Potsdam (Golm, Germany). Excitation wavelength was 488 nm. Before measurements buffer with pH = 9.2 has been added to enhance signal from fluorescein.

The catalytic reaction conversions were followed by UV-vis spectrometry on the Cary-50 spectrophotometer (Varian, Inc, Germany).

### Catalysis experiment.

Both types of silver-containing capsules were investigated for their catalytic activity in the reaction of 4-nitrophenol (4-NP) reduction into 4-amonophenol (4-AP) before or after the ultrasound was applied.

For reaction 1 mL of 0.4 M NaBH₄ and 0.05 M NaOH water solution was mixed with 25 µL of 2.4x10⁻³ M 4-NP water solution. After addition of 100 µL of capsule probe, the total volume of the sample was adjusted to 4 mL. The progress of the reaction was monitored by decrease of 4-NP peak at 400 nm. UV-vis spectra were taken each five minutes after capsules addition.

### 2.4 Results and discussion

### Continuous sonication.

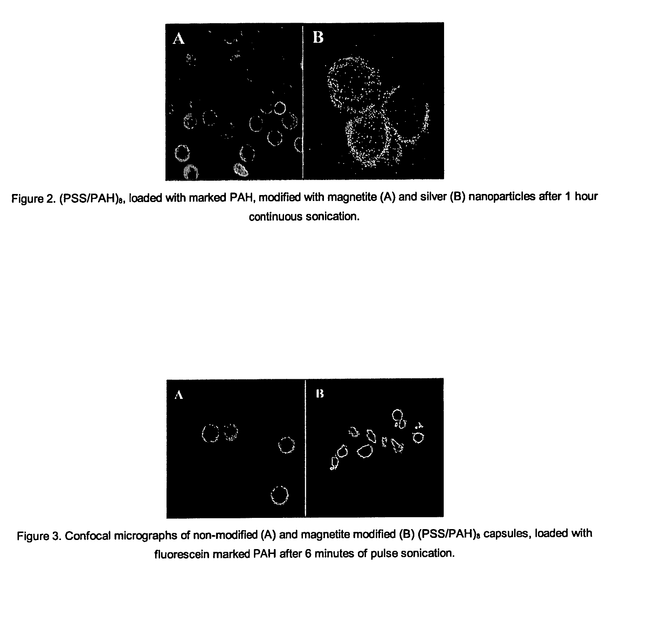

(PSS/PAH)₈ loaded with FITC-PAH and modified with silver and magnetite were tested for this experiments. Confocal microscopy data shows that even 1 hour of continuous sonication is not enough for destruction of capsules with magnetite (Figure 2A). However, if the capsule surface is modified with a continuous layer of Ag particles, sonication for more than 1 hour leads to destruction of capsules and release of their content (Figure 2B). The magnetite insencitivity to the sonication is either because of the low magnetite content, or due to the small size of the magnetite particles.

### Pulse sonication.

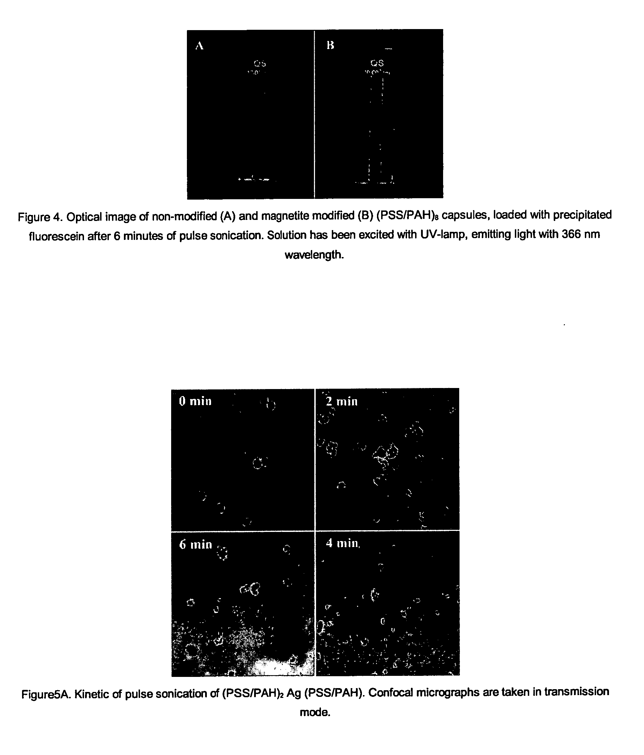

Because of time consuming and low power of continuous sonication the pulse sonication method with a sonic rod has been tested. Preliminary tests with non-modified and magnetite modified (PSS/PAH)₈ capsules loaded with fluorescein marked PAH (Figure 3) and precipitated fluorescein (Figure 4) unambiguously show influence of magnetite layer on capsule stability towards sonication. In case of PAH loaded capsules one can see pieces of capsules (Figure 3), which remain fluorescent probably due to PAH attached to them. Transformation of polyelectrolyte inside of capsules and its ability to transfer from broken ones to media is topic for separate research project. The fluorescence of solution with magnetite modified capsules (Figure 4), loaded with fluoroscein is due to dissolving of dye from capsules, broken during sonication.

### Kinetics of pulse sonication.

The influence of the sonication time on the capsules integrity was investigated for the (PSS/PAH)₂ Ag (PSS/PAH)₂ capsules and magnetite-containing capsules. Figure 5A shows the confocal transmission micrographs of the capsules treated with ultrasound for different time. One can see that more than 50 % of the capsules are broken after only 2 minutes of sonication. Four minutes of sonication leads to the complete breakage of the capsules -only pieces of the broken capsules are visible (Figure 5A).

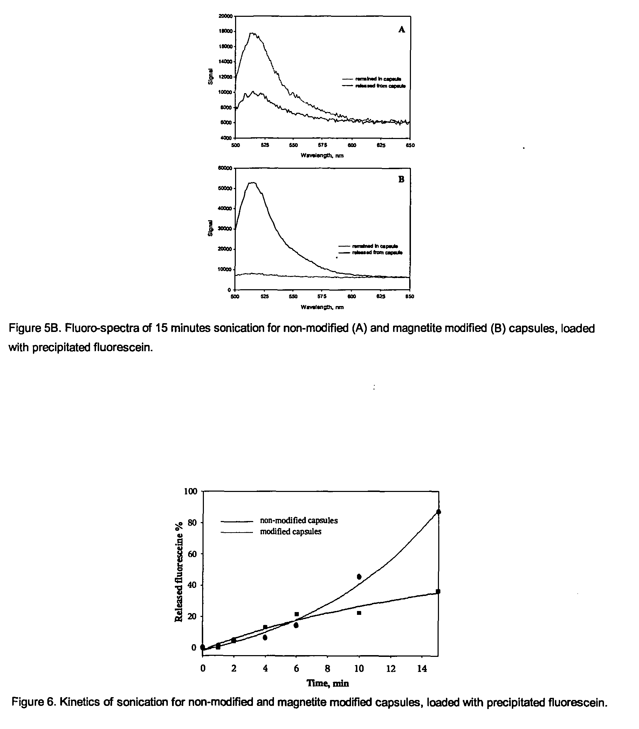

For the quantitative study of the pulse sonication kinetics fluorescein loaded (PSS/PAH)₈ capsules either non-modified or modified with magnetite or silver particles, were chosen. The ultrasound was applied for 0, 1, 2, 4, 6, 10 and 15 minutes. Sonication for 15 minutes lead to the release of 36 % of fluorescein from non-modified capsules. Contrary to that, 87 % of the dye was released in the case of the magnetite modified capsules (Fig. 5B).

The study of the dependence of the amount of dye released vs. time of sonication (Figure 6) indicates greater release of fluorescein from the magnetite modified capsules after 7 minutes of sonication. However, numerically lower amount of fluorescence from modified capsules after 4 and 6 minutes of sonication can be attributed to the influence of magnetite particles in solution, which tend to decrease overall signal from solution.

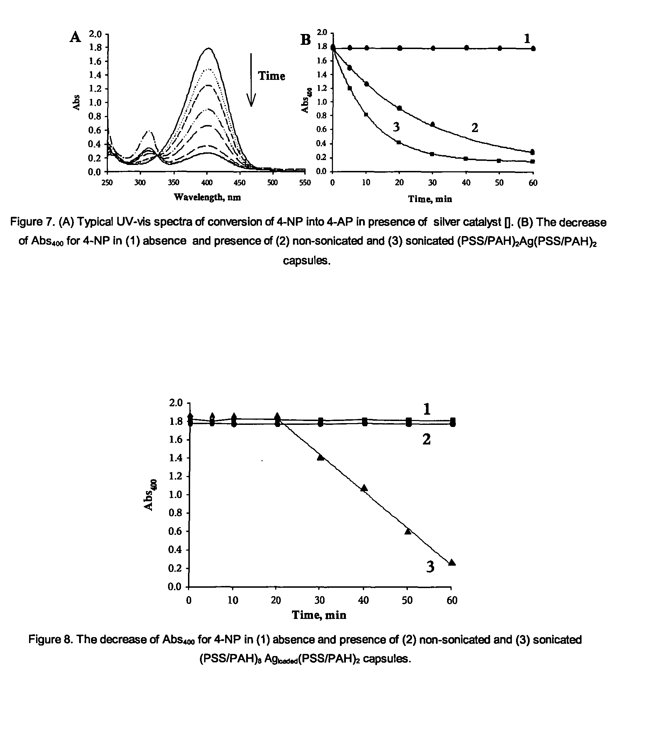

**2.5 Applicability of the method in controlled catalysis**. The reaction of reduction of 4-nitrophenol (4-NP) into 4-amonophenol (4-AP) in a presence of sodium borohydride is catalyzed by silver. The mechanism of this reaction involves the oxidation of Ag⁰ into Ag⁺ by 4-NP followed by reduction by NaBH₄. Reaction was monitored by decrease of 4-NP UV-vis peak at 400nm in the presence of a silver catalyst (Figure 7A).

***(PSS*****/*****PAH)***_{***2***} ***Ag (PSS*****/*****PAH)***_{***2***} ***capsules.*** As was shown above, 2 minute of pulse sonication is sufficient to destroy capsules. 40 µl of *(PSS*/*PAH)*_{*2*} Ag *(PSS*/*PAH)*_{*2*} capsule stock solution of capsules has been diluted with 1 mL of water. Half of the solution was sonicated in a pulse mode for 2 minutes. 100 µL of sonicated and non-sonicated capsules has been added to catalytic reaction mixture. Silver inside the non-treated capsules can catalyze the reaction (Figure 7B, line 2), however, if the sonicated capsules are added, the reaction rate becomes higher (Figure 7B, line 3). When no silver catalyst is added, conversion of 4-NP into 4-AP does not take place (Figure 7B, line 1).

The exponential decay of Abs₄₀₀ with time indicates second order of reaction of conversion (reaction depends only on concentration of only one substrates of reaction).

***(PSS*****/*****PAH)***_{***8***} ***Ag***_{***loaded***}***(PSS*****/*****PAH)***_{***2***} ***capsules*****.** Capsules after preparation have been diluted with 2 mL of water. 1 mL of obtained solution has been sonicated in a pulse mode for 6 minutes. 100 µL of sonicated and non-sonicated capsules have been added to the reagents mixture (Figure 8). As one can see, addition of non-sonicated capsules to reaction media does not catalyze reaction and no decay can bee seen (Figure 8, line 2). Addition of the sonicated capsules does not lead to any change in reaction rate for the first 20 minutes. The linear decay of 4-NP concentration starts only after 20 minutes (Figure 8, line 3).

The linear decay of 4-NP concentration indicates zero-order of catalytic reaction.

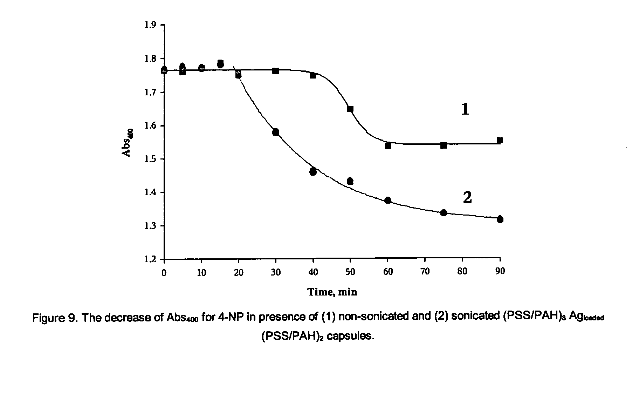

The existence of the "activation" time before silver starts to catalyze the reaction can be attributed to two factors: (a) formation of a gel-like structure by PSS inside of the capsule leads to the slower release of silver from the capsules, and (b) the surface of silver particles in the capsule interior is oxidized and the reduction of silver oxide by borohydride is limited due to the complexation of silver surface with PSS. This may also lead to the limited diffusion of the reaction reagents to the silver particles surface. To answer this question, the experiment was repeated with a higher amount of sonicated and non-sonicated capsules (950 µL vs. 200 µL, Figure 9), however, the solution of sonicated capsules has been aged for one hour before addition to the reaction mixture. However, even in this case, the 20 minutes "activation" period remains (Figure 9, line 2). This means that the reaction starts only when the surface of the silver particles can get reduced with borohydride and is no longer trapped with the PSS molecules.

The decay of 4-NP concentration in the case of higher silver quantity has an exponential shape (1^{st} order of reaction).

In addition, it was notices that conversion of 4-NP in the presence of non - sonicated capsules takes place (Figure 9, line 1). However, in this case, the "activation" period is twice longer, which indicates that the silver particles - PSS complex is more stable in time. Upon leaving overnight, reaction was completely finished in both cases (no color in reaction mixture and no peak in UV-vis adsorption spectra at 400 nm).

### References:

1. Radtchenko, I.L., et al., *Assembly of alternated multivalent ion*/*polyelectrolyte layers on colloidal particles. Stability of the multilayers and encapsulation of macromolecules into polyelectrolyte capsules.* Journal of Colloid and Interface Science, 2000. **230**(2): p. 272-280.
2. Antipov, A.A., et al., *Fabrication of a novel type of metallized colloids and hollow capsules.* Langmuir, 2002. **18**(17): p. 6687-6693.

## Claims

1. A method for remote control release of a substance from a capsule, wherein
(i) a capsule containing a substance to be released is provided,
(ii) the capsule shell is rendered permeable to the substance by forces exerted by a remote external source such that
(iii) the substance is released from the capsule.

2. The method according to claim 1, wherein the substance contained in the capsule is encapsulated in the interior of the capsule, embedded within the shell of the capsule and/or attached to the shell of the capsule.

3. The method according to claim 1 or 2, wherein a capsule is employed which is obtainable by layer-by-layer assembly of alternately charged polyelectrolytes and/or nanoparticles.

4. The method according to any of the preceding claims, wherein in step (ii) the capsule shell is opened and/or the capsule is destroyed.

5. The method according to any of the preceding claims, wherein the force exterted by a remote external force is selected from ultrasound or/and light.

6. The method of claim 5, wherein the remote release is caused by ultrasound selected from continuous sonication or/and pulse sonication.

7. The method according to claim 5, wherein light having near-ultraviolet, visible or/and infrared wavelengths is used.

8. The method according to claim 5 or 7, wherein the remote release is caused by light irradiation selected from continuous wave irradiation or/and pulsed irradiation.

9. The method of claim 5, 7 or 8, wherein the light irradiation is effected by a laser.

10. The method according to any of claims 5 or 7-9, wherein light having a wavelength from 700 to 1000 nm is irradiated.

11. The method according to any of the preceding claims, wherein the forces exerted by an external source have no impact on the substance to released.

12. The method according to any of the preceding claims, wherein the capsule shell is doped with material sensitive and/or susceptible to the force exerted by the external source.

13. The method according to claim 11, wherein the shell is doped with Ag nanoparticles, dyes, magnetite nanoparticles, nanodiamonds, silica nanoparticles, polymers and/or ceramic nanoparticles.

14. The method according to any of the preceding claims, wherein a label, in particular, a fluorescence label, is used to monitor substance release.

15. The method according to any of the preceding claims, wherein the substance to be released is selected from drugs, biomolecules, catalytically active molecules and/or biosensing molecules.

16. A capsule for remote control release comprising a capsule shell obtainable by layer-by-layer assembly of alternately charged polyelectrolytes and/or nanoparticles, wherein the shell is doped with material sensitive and/or susceptible to a force exerted by an remote external source, which force renders the capsule shell wall permeable to a substance to be released.

17. The capsule according to claim 15, containing a substance to be released encapsulated, embedded within the capsule shell or/and attached to the shell of the capsule.

18. Use of a method according to any of claims 1 to 14 or of a capsule according to claim 15 or 16 in catalysis, biotechnology, biosensing and/or medicine.
